# EUROPÄISCHE PATENTSCHRIFT

(11) **EP 0 564 405 B1**
(45) Veröffentlichungstag und Bekanntmachung des Hinweises auf die Patenterteilung: **16.07.1997**
(21) Anmeldenummer: 93810209.2
(22) Anmeldetag: 23.03.1993
(51) Int. Cl.: C07D 309/12, C07D 307/20, G03F 7/00

(54) **Phenylessigsäureester und ihre Anwendungen**
Phenylacetic acid esters and their applications
Esters d'acide phénylacétique et leurs applications

(30) Priorität: 01.04.1992 CH 1054/92
(43) Veröffentlichungstag der Anmeldung: 06.10.1993
(73) Patentinhaber: OCG Microelectronic Materials Inc., West Paterson New Jersey 07424 (US)
(72) Erfinder: Steinmann, Alfred, Dr., CH-1724 Praroman (CH)
(74) Vertreter: Klunker . Schmitt-Nilson . Hirsch

(56) Entgegenhaltungen:
- EP-A- 0 298 300
- EP-A- 0 367 132
- JOURNAL OF ORGANIC CHEMISTRY, Band 43, Nr. 18, 1978, Easton, PA (US); C. KRUSE et al., Seiten 3548-3552

## Beschreibung

Gegenstand der vorliegenden Erfindung sind neue Phenylessigsäureester, Zusammensetzungen enthaltend diese Phenylessigsäureester, ein Verfahren zur Herstellung von Abbildungen unter Verwendung dieser Ester, sowie die Verwendung der Ester als Lösungsinhibitoren und der besagten Zusammensetzungen als Positiv-Resists.

Bei den wässrig entwickelbaren Photoresists wird bekanntlich ein filmbildendes, wässrig oder wässrig-alkalisch lösliches Harz mit einem Lösungsinhibitor (dissolution inhibitor) gemischt, der die Löslichkeit des Gemisches in einem wässrig-alkalischen Entwickler stark herabsetzt. Durch eine photochemische Reaktion verändert sich der Lösungsinhibitor derart, zum Beispiel durch die Depolymerisation, Acetalspaltung, Orthoesterspaltung oder Bildung einer Carbonsäure aus einem o-Chinondiazid, dass der Photoresist an den belichteten Stellen in dem Entwickler wieder gut löslich ist.

Bekannte Lösungsinhibitoren für positive Photoresists sind beispielsweise 1,2-Naphthochinondiazide (vgl. J. Kosar in "Light-Sensitive Systems" Verlag John Wiley & Sons, New York 1965, Seite 339 bis 352) in Novolakharzen. 1,2-Naphthochinondiazide sind photoaktiv und wirken an unbelichteten Stellen im Photoresist als Lösungsinhibitoren. Jedoch absorbieren sie sehr stark im DUV-Bereich (DUV: **d**eep **u**ltra-**v**iolet; Bereich zwischen 200 und 300 nm) und können somit nur sehr beschränkt in der DUV-Lithographie eingesetzt werden.

In EP-A 367 131 und EP-A 367 132 werden beispielsweise strahlungsempfindliche Gemische beschrieben, die im wesentlichen bestehen aus (a) einem in wässrig-alkalischen Lösungen löslichen Bindemittel, (b) einer bei Bestrahlung eine starke Säure bildenden Verbindung und (c) einem die Löslichkeit von (a) inhibierenden Malonsäureester bzw. β-Ketosäureester. Für diese strahlungsempfindlichen Gemische wird eine hohe Bestrahlungsenergie benötigt. Weiterhin werden diese Gemische mit stark alkalischen Lösungen entwickelt.

C. G. Kruse et al. beschreiben in J. Org. Chem. 43, 3548 (1978) den Einsatz von (2-Tetrahydrofuranyl)diphenylacetat und von (2-Tetrahydropyranyl)diphenylacetat als Ausgangsprodukte zur Herstellung von Tetrahydrofuranyl- bzw. Tetrahydropyranylethern.

Es wurde nun gefunden, dass (2-Tetrahydrofuranyl)diphenylacetat und (2-Tetrahydropyranyl)diphenylacetat sowie bestimmte andere Phenylessigsäureester wertvolle Lösungsinhibitoren in strahlungsempfindlichen, positiv arbeitenden Photoresistsystemen darstellen. Diese Photoresists weisen eine hohe Empfindlichkeit gegenüber aktinischen Strahlen, insbesondere im DUV-Bereich auf. Darüber hinaus bewirken sie nach der Belichtung grosse Löslichkeitsunterschiede zwischen belichteten und unbelichteten Zonen, so dass Schichten mit Submikron-Auflösung hergestellt werden können.

Gegenstand vorliegender Erfindung sind somit neue Verbindungen der Formel I 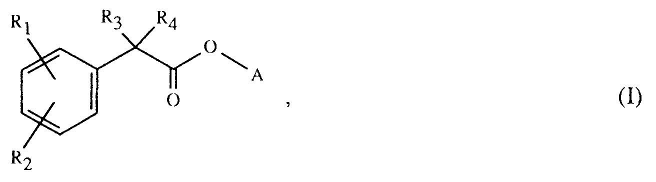 worin A einen Rest der Formeln 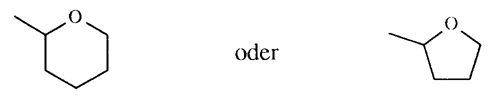 bedeutet,
R₁ und R₂ unabhängig voneinander Wasserstoff, C₁-C₄-Alkyl, C₁-C₄-Alkoxy, Halogen oder einen Rest der Formeln -CH₂-COOA oder 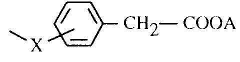 bedeuten, wobei X -O-, -CH₂-, -C(CH₃)₂- oder -SO₂- darstellt, und
R₃ und R₄ unabhängig voneinander Wasserstoff, C₁-C₄-Alkyl oder Phenyl bedeuten, wobei (2-Tetrahydrofuranyl)diphenylacetat und (2-Tetrahydropyranyl)diphenylacetat ausgeschlossen sind.

Bevorzugte Verbindungen der Formel I sind solche, worin R₂ Wasserstoff bedeutet.

Weiterhin bevorzugt sind Verbindungen der Formel I, worin R₁ Wasserstoff, Halogen oder einen Rest der Formel -CH₂-COOA bedeutet, und R₃ und R₄ unabhängig voneinander Wasserstoff oder Phenyl bedeuten.

Insbesondere bevorzugt sind Verbindungen der Formeln 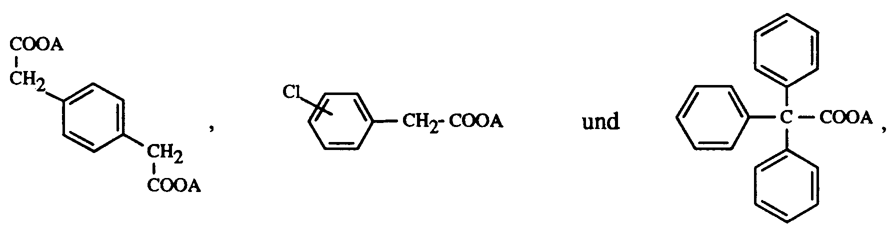 worin A die obige Bedeutung hat.

Stellt ein Substituent C₁-C₄-Alkyl dar, so kann dieser gradkettig oder verzweigt sein. Beispielsweise handelt es sich um Methyl, Ethyl, n-Propyl, Isopropyl, n-Butyl, sec-Butyl, Isobutyl oder tert.-Butyl.

Als C₁-C₄-Alkoxysubstituenten kommen beispielsweise Methoxy, Ethoxy, n-Propoxy oder n-Butoxy in Frage.

Bedeutet ein Substituent Halogen, so handelt es sich um Fluor, Chlor, Brom oder Jod, bevorzugt um Chlor und Brom, insbesondere aber um Chlor.

Die Verbindungen der Formel I können beispielsweise hergestellt werden, indem man eine Verbindung der Formel II 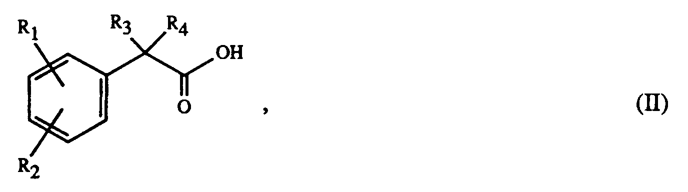 worin R₁, R₂, R₃ und R₄ die oben angegebene Bedeutung haben, mit 2,3-Dihydrofuran oder 3,4-Dihydro-2H-pyran in einem sauren Medium in an sich bekannter Weise zu Verbindungen der Formel I umsetzt.

Die Verbindungen der Formel II stellen an sich bekannte Verbindungen dar und sind teilweise im Handel erhältlich. Beispiele hierfür sind 2-, 3- oder 4-Chlorphenylessigsäure, 1,4-Phenylendiessigsäure oder Triphenylessigsäure.

2,3-Dihydrofuran und 3,4-Dihydro-2H-pyran sind ebenfalls im Handel erhältlich.

Das saure Medium der Reaktionslösung kann beispielsweise dadurch hergestellt werden, dass man zur Reaktionslösung einige Tropfen z.B. einer anorganischen Säure, wie konzentrierte Salzsäure oder Schwefelsäure, zugibt.

Die Reaktionstemperatur bewegt sich zweckmässig im Bereich von 10 bis 150 °C, vorzugsweise zwischen 30 und 120 °C, besonders bevorzugt zwischen 30 und 90 °C. Im allgemeinen wird die Reaktion ohne Zusatz eines Lösungsmittels durchgeführt. Gegebenenfalls kann aber ein Lösungsmittel anwesend sein, das unter den Reaktionsbedingungen inert sein muss. Als Lösungsmittel kommen u.a. aromatische Kohlenwasserstoffe, chlorierte Kohlenwasserstoffe, Ketone und Ether in Betracht. Beispiele dafür sind Benzol, Toluol, Xylol, Ethylbenzol, Isopropylbenzol, Ethylenchlorid, Propylenchlorid, Methylenchlorid, Chloroform, Methylethylketon, Aceton, Cyclohexanon, Diethylether und Tetrahydrofuran.

Wie eingangs erwähnt, zeigen die erfindungsgemässen Phenylessigsäureester der Formel I lösungsinhibierende Eigenschaften und stellen somit wertvolle Materialien für positiv arbeitende Photoresists dar. Diese Ester weisen eine sehr gute inhibierende Wirkung in üblicherweise verwendeten Bindemitteln, wie Novolake oder Poly-(hydroxystyrole), auf und bilden dann zusammen mit säuregenerierenden Photoinitiatoren ein strahlungsempfindliches Gemisch, das saukturiert werden kann: Durch aktinische Bestrahlung zerfällt der Säuregenerator und die gebildete Säure spaltet die erfindungsgemässen Ester katalytisch. Die so entstandenen Phenylessigsäurederivate sind in wässrig-alkalischen Lösungen gut löslich. Folglich können in dem Gemisch die belichteten Zonen herausgelöst werden, während die unbelichteten Zonen unlöslich bleiben.

Die Umwandlung der erfindungsgemässen Ester zu Carbonsäuren kann bei sehr kleinen Belichtungsdosen erfolgen. Desweiteren ist der Polaritätsunterschied zwischen Ester und korrespondierender Säure sehr gross. Das führt zu grossen Löslichkeitsunterschieden zwischen belichtetem und unbelichtetem Photoresistmaterial. Dieses grosse Kontrastverhalten des Resists fördert die Auflösung von Submikronstrukturen.

Die Erfindung umfasst somit auch positiv arbeitende, strahlungsempfindliche Zusammensetzungen enthaltend, bezogen auf die Gesamtmenge der Komponenten A), B) und C),
A) 55 bis 95 Gew.% mindestens eines in wässrig-alkalischer Lösung löslichen Bindemittels,
B) 4,5 bis 40 Gew.% mindestens einer Verbindung der Formel I und
C) 0,5 bis 15 Gew.% mindestens einer unter Einwirkung aktinischer Strahlung säurebildenden Substanz.

Gegenstand der Erfindung ist ferner die Verwendung der Verbindungen gemäss Formel I als Lösungsinhibitoren in Photoresist-Zusammensetzungen.

Bevorzugt sind Zusammensetzungen enthaltend, bezogen auf die Gesamtmenge der Komponenten A), B) und C),
A) 65 bis 90 Gew.% mindestens eines in wässrig-alkalischer Lösung löslichen Bindemittels,
B) 9 bis 30 Gew.% mindestens einer Verbindung der Formel I und
C) 1 bis 10 Gew.% mindestens einer unter Einwirkung aktinischer Strahlung säurebildenden Substanz.

Die Wahl des Bindemittels erfolgt je nach dem Anwendungsgebiet und den hierfür geforderten Eigenschaften, wie Entwickelbarkeit in wässrig-alkalischen Lösungsmittelsystemen, Adhäsion auf den Substraten oder das Absorptionverhalten.

Als Komponente A) der erfindungsgemässen Zusammensetzungen wird bevorzugt ein phenolisches Harz eingesetzt.

Insbesondere bevorzugt sind Zusammensetzungen, worin als Komponente A) ein Novolak, ein Poly-(4-hydroxystyrol), Poly-(4-hydroxy-α-methylstyrol), Copolymere von N-(4-Hydroxyphenyl)-maleinimid mit Styrol, 4-Hydroxystyrol, Acrylsäure und Metharylsäure oder Copolymere von 4-Hydroxystyrol und Alkoxystyrol eingesetzt werden.

Ganz besonders bevorzugt sind Zusammensetzungen, worin als Komponente A) ein Poly-(4-hydroxystyrol) eingesetzt wird.

Handelt es sich bei der Komponente A) um einen Novolak, so leitet sich dieser beispielsweise von einem Aldehyd, vorzugsweise Formaldehyd, Acetaldehyd oder Furfuraldehyd, besonders jedoch von Formaldehyd, und einem Phenol ab. Die phenolische Komponente ist vorzugsweise Phenol selbst, oder auch halogeniertes Phenol, beispielsweise substituiert mit ein oder zwei Chloratomen, vorzugsweise p-Chlorphenol, oder sie ist ein durch ein oder zwei C₁-C₉-Alkylgruppen substituiertes Phenol, beispielsweise o-, m- oder p-Kresol, ein Xylenol, p-tert.-Butylphenol oder p-Nonylphenol. Es kann sich bei der Phenolkomponente der bevorzugten Novolake aber auch um p-Phenylphenol; Resorcin, Bis-(4-hydroxyphenyl)-methan oder 2,2-Bis-(4-hydroxyphenyl)-propan handeln.

Den alkalilöslichen Bindemitteln gemäss Komponente A) können gegebenenfalls noch weitere Zusatzharze beigegeben werden, wie bei den Positivsystemen auf Diazoketon-Basis üblich. Zu diesen Zusatzharzen zählen beispielsweise Vinylpolyinerisate, wie Polyvinylacetat, Polyacrylate, Polyvinylether oder Polyvinylpyrrolidone. Im allgemeinen werden jedoch nicht mehr als 20 Gew.%, bezogen auf die Menge an alkalilöslichem Bindemittel, von diesen Zusatzharzen zugefügt.

Als strahlungsempfindliche Komponenten B), die bei Lichteinwirkung Säuren bilden bzw. abspalten, sind eine grosse Anzahl von Verbindungen bekannt. Dazu zählen beispielsweise Diazoniumsalze, wie sie in der Diazotypie verwendet werden, o-Chinondiazide, wie sie in bekannten positiv arbeitenden Kopiermassen verwendet werden, oder auch Halogenverbindungen, die bei Bestrahlung Halogenwasserstoffsäure bilden. Verbindungen dieses Typs sind beispielsweise in US-A 3,515,552, 3,536,489 oder 3,779,778, sowie in DE-A 27 18 259, 22 43 621 oder 26 10 842 beschrieben.

Als strahlungsempfindliche Komponenten B) der erfindungsgemässen Zusammensetzungen eignen sich vor allem kationische Photoinitiatoren aus der Gruppe der Iodonium- oder Sulfoniumsalze. Solche Verbindungen sind beispielsweise in "UV-Curing, Science and Technology" (Editor: S.P. Pappas, Technology Marketing Corp., 642 Westover Road, Stanford, Connecticut, USA) beschrieben.

Ferner können Sulfoxoniumsalze als strahlungsempfindliche Verbindungen verwendet werden. Solche Salze sind beispielsweise in der EP-B 35,969 oder in der EP-A 44,274 bzw. 54,509 beschrieben. Insbesondere zu erwähnen sind aliphatische Sulfoxoniumsalze, die im tiefen UV-Bereich absorbieren.

Es lassen sich auch Verbindungen einsetzen, die bei Bestrahlung mit aktinischem Licht Sulfonsäuren freisetzen. Solche Verbindungen sind an sich bekannt und beispielsweise in der GB-A 2,120,263, in den EP-A 84,515, 37,152 oder 58,638 bzw. in den US-A 4,258,121 oder 4,371,605 beschrieben.

Werden als strahlungsempfindliche säureabspaltende Komponenten B) Salze eingesetzt, so sind diese vorzugsweise in organischen Lösungsmitteln löslich. Besonders bevorzugt handelt es sich bei diesen Salzen um Produkte mit komplexen Säuren, beispielsweise von Borfluorwasserstoffsäure, Hexafluorphosphorsäure, Trifluormethansulfonsäure, Hexafluorarsensäure oder Hexafluorantimonsäure.

Die erfindungsgemässen Zusatz ensetzungen können weiterhin übliche Zusatzstoffe enthalten, wie z.B. Stabilisatoren, Pigmente, Farbstoffe, Füllstoffe, Haftvermittler, Verlaufsmittel, Netzmittel und Weichmacher. Ferner können die Zusatz ensetzungen zur Applikation in geeigneten Lösungsmitteln gelöst werden.

Die erfindungsgemässen Zusammensetzungen eignen sich hervorragend als Beschichtungsmittel für Substrate aller Art, z.B. Holz, Textilien, Papier, Keramik, Glas, Kunststoffe, wie Polyester, Polyethylenterephthalat, Polyolefine oder Celluloseacetat, insbesondere in Form von Filmen, sowie Metalle, wie Al, Cu, Ni, Fe, Zn, Mg oder Co, und GaAs, Si oder SiO₂, bei denen durch bildmässiges Belichten eine Abbildung aufgebracht werden soll.

Ein weiterer Gegenstand der vorliegenden Erfindung ist daher ein Verfahren zur Herstellung von positiven Abbildungen durch
I) Beschichtung eines Substrates mit einer erfindungsgemässen strahlungsempfindlichen Zusammensetzung,
II) Belichtung des beschichteten Substrates mit einem vorbestimmten Muster aktinischer Strahlung und
III) Entwicklung des belichteten Substrates.

Die Beschichtung der Substrate kann z.B. erfolgen, indem man eine Lösung oder Suspension der erfindungsgemässen Zusammensetzung auf das Substrat appliziert.

Die Wahl des Lösungsmittels und die Konzentration richtet sich hauptsächlich nach der Art der Zusammensetzung und nach dem Beschichtungsverfahren. Die Lösung wird mittels bekannter Beschichtungsverfahren auf ein Substrat gleichförmig aufgebracht, z.B. durch Schleudern, Tauchen, Rakelbeschichtung, Vorhanggiessverfahren, Aufpinseln, Sprühen, insbesondere durch elektrostatisches Sprühen und Reverse-Rollbeschichtung. Es ist auch möglich, die strahlungsempfindliche Schicht auf einen temporären, flexiblen Träger zu bringen, und dann durch Schichtübertragung via Lamination das endgültige Substrat, z.B. eine kupferkaschierte Leiterplatte, zu beschichten.

Die Auftragsmenge (Schichtdicke) und Art des Substrates (Schichtaäger) sind abhängig vom gewünschten Applikationsgebiet. Besonders vorteilhaft ist es, dass die erfindungsgemässen Zusammensetzungen in sehr variablen Schichtdicken eingesetzt werden können. Dieser Schichtdickenbereich umfasst Werte von ca. 0,5 µm bis mehr als 100 µm.

Nach dem Beschichten wird das Lösungsmittel in der Regel durch Trocknen entfernt, und es resultiert eine Schicht des Photoresists auf dem Träger.

Nach der in üblicher Weise erfolgten bilmässigen Belichtung des Materials werden die belichteten Stellen des Photolackes durch Herauslösen in einem Entwickler entfernt.

Der Begriff 'bildmässige' Belichtung beinhaltet sowohl die Belichtung durch eine Photomaske, die ein vorbestimmtes Muster enthält, beispielsweise ein Diapositiv, die Belichtung durch einen Laserstrahl, der beispielsweise computergesteuert über die Oberfläche des beschichteten Substrates bewegt wird und auf diese Weise ein Bild erzeugt, sowie die Bestrahlung mit computergesteuerten Elektronenstrahlen oder die Behandlung mit Röntgenstrahlen durch eine Absorbermaske hindurch.

Die Lichtempfindlichkeit der erfindungsgemässen Zusammensetzungen reicht in der Regel vom UV-Gebiet (ca. 200 nm) bis ca. 600 nm und umspannt somit einen sehr breiten Bereich. Insbesondere im DUV-Bereich (200-300 nm) weisen die erfindungsgemässen Zusatz ensetzungen eine ausgezeichnete Transparenz auf. Als Lichtquellen kommen daher eine grosse Anzahl der verschiedensten Typen zur Anwendung. Es sind sowohl Punktlichtquellen als auch flächenförmige Strahler (Lampenteppiche) geeignet. Beispiele sind: Kohlelichtbogenlampen, Xenon-Lichtbogenlampen, Quecksilberdampflampen, gegebenenfalls mit Metall-Halogeniden dotiert (Metall-Halogenlampen), Fluoreszenzlampen, Argonglühlampen, Elektronenblitzlampen, photographische Flutlichtlampen, Elektronenstrahlen und Röntgenstrahlen. Der Abstand zwischen Lampe und erfindungsgemässem Bildmaterial kann je nach Anwendungszweck und Lampentyp bzw. -stärke variieren, z.B. zwischen 2 cm bis 150 cm. Speziell geeignet sind Laserlichtquellen, z.B. Argonionenlaser oder Kryptonionenlaser. Bei dieser Art der Belichtung ist eine Photomaske nicht mehr unbedingt nötig; der gesteuerte Laser-Strahl schreibt direkt auf die Schicht. Hier ist die hohe Empfindlichkeit der erfindungsgemässen Materialien sehr vorteilhaft, die hohe Schreibgeschwindigkeiten bei relativ niedrigen Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Die Wahl des jeweiligen Entwicklers richtet sich nach der Art des Photolackes oder der entstehenden Photolyseprodukte. Der Entwickler kann wässrige Lösungen von Basen umfassen, denen gegebenenfalls organische Lösungsmittel oder deren Mischungen zugesetzt werden.

Besonders bevorzugt als Entwickler werden wässrig-alkalische Lösungen, wie sie auch für die Entwicklung von Naphthochinondiazidschichten eingesetzt werden. Dazu zählen insbesondere wässrige Lösungen von Alkalimetallsilikaten, -phosphaten, -hydroxiden, - carbonaten und -hydrogencarbonaten. Ebenfalls bevorzugt werden Metallionen-freie Entwickler, wie beispielsweise wässrige Tetramethylammoniumhydroxid-Lösungen. Diesen Lösungen können gegebenenfalls noch kleinere Mengen an Netzmitteln und/oder organischen Lösungsmitteln zugesetzt sein.

Typische organische Lösungsmittel, die den Entwicklerflüssigkeiten zugesetzt werden können, sind beispielsweise Cyclohexanon, 2-Ethoxyethanol, Toluol, Aceton, sowie Mischungen zweier oder mehrerer dieser Lösungsmittel.

Mögliche Einsatzgebiete der erfindungsgemässen Zusammensetzungen sind die Verwendung als Photoresists für die Elektronik (Galvanoresist, Ätzresist, Lötstoppresist), die Herstellung von Druckplatten, wie Offsetdruckplatten oder Siebdruckformen, der Einsatz beim Formteilätzen oder der Einsatz als Mikroresist bei der Herstellung integrierter Schaltkreise. Dementsprechend unterschiedlich sind die möglichen Schichtträger und die Verarbeitungsbedingungen der beschichteten Substrate.

Für photographische Informationsaufzeichnung dienen z.B. Folien aus Polyester, Celluloseacetat oder mit Kunststoff beschichtete Papiere; für Offsetdruckformen speziell behandeltes Aluminium, für die Herstellung gedruckter Schaltungen kupferkaschierte Laminate und für die Herstellung von integrierten Schaltkreisen Siliziumwafer. Die Schichtdicken für photographische Materialien und Offsetdruckformen betragen ca. 0,5 µm bis 10 µm; für gedruckte Schaltungen 1 µm bis ca. 100 µm und für integrierte Schaltkreise 0,5 µm bis 2 µm.

Gegenstand der Erfindung ist daher die Verwendung der erfindungsgemässen Zusatz ensetzungen als Positiv-Resist für die Herstellung von Druckformen, gedruckten Schaltungen oder integrierten Schaltkreisen, sowie für silberlose photographische Filme.

Die Erfindung betrifft weiterhin auch die unter Verwendung der erfindungsgemässen Zusammensetzungen hergestellten Druckformen, gedruckten Schaltungen, integrierten Schaltkreise oder silberlosen photographischen Filme.

Die folgenden Beispiele illustrieren die vorliegende Erfindung weiter.

### I. Herstellungsbeispiele

### Beispiel A: Synthese von 4-Chlorphenylessigsäure-tetrahydrofuran-2-ylester

34 g (200 mmol) 4-Chlorphenylessigsäure werden in 42 g (600 mmol) 2,3-Dihydrofuran aufgeschlämmt und mit 2 Tropfen konzentrierter Salzsäure versetzt. Es tritt sofort eine exotherme Reaktion ein und das Reaktionsgemisch wird klar. Nach dem Abklingen der Reaktion wird das Gemisch noch 30 Minuten bei 40 °C gerührt. Anschliessend wird die Lösung auf eine eiskalte NaHCO₃-Lösung gegossen. Danach wird das Produkt mit Diethylether extrahiert. Die organische Phase wird mit Na₂SO₄ getrocknet und filtriert. Das Lösungsmittel wird dann am Rotationsverdampfer abgedampft. Die verbleibende Müssigkeit wird bei 40 °C mit n-Hexan aufgenommen. Anschliessend lässt man das Produkt bei 0 °C auskristallisieren. Die erhaltenen Kristalle schmelzen bei 41 °C.
Ausbeute: 37,8 g (77 % der Theorie).

| | | | | |
|---|---|---|---|---|
| Elementaranalyse: | ber.: | *C* 59,88 %; | *H* 5,44 %; | *Cl* 14,73 %. |
| | gef.: | *C* 59,84 %; | *H* 5,42 %; | *Cl* 14,65 %. |

¹H-NMR (CDCl₃): 1,81-2,08 ppm, m (-C**H**₂-C**H**₂-); 3,56 ppm, s (Aryl-C**H**₂-CO-); 3,79-4,05 ppm, m (-C**H**₂-O-); 6,27-6,30 ppm, m (-O-C**H**-O-); 7,14-7,37 ppm, m (Aryl-**H**).

### Beispiel B: Synthese von 4-Chlorphenylessigsäure-tetrahydropyran-2-ylester

30 g (176 mmol) 4-Chlorphenylessigsäure werden mit 42 g (500 mmol) 3,4-Dihydro-2H-pyran vermischt und mit 3 Tropfen konzentrierter Salzsäure versetzt. Es tritt sofort eine exotherme Reaktion ein und das Reaktionsgemisch wird klar. Nach dem Abklingen der Reaktion wird noch 30 Minuten bei 40 °C gerührt. Anschliessend wird die Lösung auf eine eiskalte NaHCO₃-Lösung gegossen. Danach wird das Produkt mit Diethylether extrahiert. Die organische Phase wird mit Na₂SO₄ getrocknet und filtriert. Das Lösungsmittel wird dann am Rotationsverdampfer abgedampft. Die verbleibende Flüssigkeit wird bei 40 °C mit n-Hexan aufgenommen. Anschliessend lässt man das Produkt bei 0 °C auskristallisieren. Die erhaltenen Kristalle schmelzen bei 66 °C.
Ausbeute: 30,5 g (68 % der Theorie).

| | | | | |
|---|---|---|---|---|
| Elementaranalyse: | ber.: | *C* 61,30 %; | *H* 5,94 %; | *Cl* 13,92 %. |
| | gef.: | *C* 61,41 %; | *H* 5,90 %; | *Cl* 13,86 %. |

¹H-NMR (CDCl₃): 1,45-1,90 ppm, m (-C**H**₂-C**H**₂-C**H**₂-); 3,67 ppm, s (Aryl-C**H**₂-CO-); 3,63-3,78 ppm, m (-C**H**₂-O-); 5,98 ppm, s (-O-C**H**-O-); 7,17-7,36 ppm, m (Aryl-**H**).

### Beispiel C: Synthese von 1,4-Phenylendiessigsäure-di-(tetrahydropyran-2-ylester)

19,4 g (100 mmol) 1,4-Phenylendiessigsäure werden mit 84 g (1 mol) 3,4-Dihydro-2H-pyran vermischt und mit 10 Tropfen konzentrierter Salzsäure versetzt. Danach wird die Lösung bei 60 °C 3 Stunden gerührt und anschliessend analog Beispiel A aufgearbeitet. Nach dem Umkristallisieren in Cyclohexan wird ein Produkt erhalten, das bei 103 °C schmilzt.
Ausbeute: 18 g (50 % der Theorie).

| | | | |
|---|---|---|---|
| Elementaranalyse: | ber.: | *C* 66,28 %; | *H* 7,23 %. |
| | gef.: | *C* 66,33 %; | *H* 7,28 %. |

¹H-NMR (CDCl₃): 1,52-1,76 ppm, m (-C**H**₂-C**H**₂-C**H**₂-); 3,64 ppm, s (Aryl-C**H**₂-CO-); 3,61-3,81 ppm, m (-C**H**₂-O-); 5,99 ppm, s (-O-C**H**-O-); 7,27 ppm, s (Aryl-**H**).

### Beispiel D: Synthese von 3-Chlorphenylessigsäure-tetrahydropyran-2-ylester

50 g (293 mmol) 3-Chlorphenylessigsäure werden mit 75 g (892 mmol) 3,4-Dihydro-2H-pyran und 3 Tropfen konzentrierter Salzsäure versetzt und anschliessend analog Beispiel A aufgearbeitet. Es wird ein Produkt erhalten, das bei 42,5 °C schmilzt.
Ausbeute: 53 g (71 % der Theorie).

| | | | | |
|---|---|---|---|---|
| Elementaranalyse: | ber.: | *C* 61,30 %; | *H* 5,94 %; | *Cl* 13,92 %. |
| | gef.: | *C* 61,17 %; | *H* 5,87 %; | *Cl* 13,88 %. |

¹H-NMR (CDCl₃): 1,52-1,83 ppm, m (-C**H**₂-C**H**₂-C**H**₂-); 3,64 ppm, s (Aryl-C**H**₂-CO-); 3,62-3,78 ppm, m (-C**H**₂-O-); 5,99 ppm, s (-O-C**H**-O-); 7,17-7,31 ppm, m (Aryl-**H**).

### Beispiel E: Synthese von 2-Chlorphenylessigsäure-tetrahydropyran-2-ylester

50 g (293 mmol) 2-Chlorphenylessigsäure werden mit 75 g (892 mmol) 3,4-Dihydro-2H-pyran und 3 Tropfen konzentrierter Salzsäure versetzt und anschliessend analog Beispiel A aufgearbeitet. Es wird ein Produkt erhalten, das bei 38 °C schmilzt.
Ausbeute: 56,7 g (76 % der Theorie).

| | | | | |
|---|---|---|---|---|
| Elementaranalyse: | ber.: | *C* 61,30 %; | *H* 5,94 %; | *Cl* 13,92 %. |
| | gef.: | *C* 61,23 %; | *H* 5,88 %; | *Cl* 13,81 %. |

¹H-NMR (CDCl₃): 1,42-1,78 ppm, m (-C**H**₂-C**H**₂-C**H**₂-); 3,82 ppm, s (Aryl-C**H**₂-CO-); 3,57-3,88 ppm, m (-C**H**₂-O-); 6,03 ppm, s (-O-C**H**-O-); 7,17-7,44 ppm, m (Aryl-**H**).

### Beispiel F: Synthese von Triphenylessigsäure-(tetrahydropyran-2-ylester)

10 g (35 mmol) Triphenylessigsäure werden mit 8,8 g (104 mmol) 3,4-Dihydro-2H-pyran vermischt und mit 2 Tropfen konzentrirerter Salzsäure versetzt. Danach wird die Lösung bei 70 °C 3 Stunden gerührt und anschliessend analog Beispiel A aufgearbeitet. Nach dem Umkristallisieren in n-Hexan wird ein Produkt erhalten, das bei 115 °C schmilzt.
Ausbeute: 6,5 g (50 % der Theorie).

| | | | |
|---|---|---|---|
| Elementaranalyse: | ber.: | *C* 80,62 %; | *H* 6,50 %. |
| | gef.: | *C* 80,41 %; | *H* 6,48 %. |

¹H-NMR (CDCl₃): 1,15-2,81 ppm, m (-C**H**₂-C**H**₂-C**H**₂-); 3,24-3,63 ppm, m (-C**H**₂-O-); 6,22 ppm, s (-O-C**H**-O-); 7,10-7,42 ppm, m (Aryl-**H**).

### II. Applikationsbeispiele

### Beispiel 1:

2,5 g des Lösungsinhibitors gemäss Beispiel B werden zusammen mit 7,5 g Resin M® P4HS [Poly-(4-hydroxystyrol) der Fa. Maruzen Oil Comp., Japan] und 0,5 g Triphenylsulfoniumtrifluoromethansulfonat in 25 ml Cyclopentanon gelöst. Dieses Gemisch wird durch ein 0,5 µm Filter auf eine Siliciumscheibe mit einem Durchmesser von 76,2 mm aufgebracht. Durch Schleuderbeschichtung bei 3000 U/min wird ein homogener Film erzeugt, der nach dem Trocknen während 60 Sekunden bei 90 °C auf einer Heizplatte eine Schichtdicke von 0,9 µm aufweist. Durch Vakuumkontakt wird eine Maske auf den Resistfilm gelegt. Anschliessend wird der Resist durch ein 254 nm Engbandfilter belichtet. Die Belichtungsdosis beträgt 5 mJ/cm². Auf einer Heizplatte wird der Resistfilm bei 80 °C während 60 Sekunden erwärmt und anschliessend 120 Sekunden in MF 312 [wässrige Tetramethylammoniumhydroxid-Lösung der Fa. Shipley], verdünnt mit 3 Teilen Wasser, entwickelt, wobei sich die belichteten Zonen im Entwickler lösen (Positiv-Resist).
Dabei werden 0,75 µm-Linien mit nahezu senkrechtem Wandprofil erhalten.

### Beispiel 2:

1 g des Lösungsinhibitors gemäss Beispiel C werden zusammen mit 7,5 g Resin M® P4HS und 0,1 g Triphenylsulfoniumtrifluoromethansulfonat in 20 ml Cyclohexanon gelöst. Nach dem Applizieren auf eine Siliciumscheibe analog Beispiel 1 wird ein 1 µm dicker Film erhalten. Dieser Film wird bei 254 nm mit einer Belichtungsdosis von 4 mJ/cm² durch eine Maske belichtet und anschliessend während 60 Sekunden bei 90 °C erwärmt. Die so gewonnenen latenten Bilder werden in MF 312, verdünnt mit 4 Teilen Wasser, während 140 Sekunden entwickelt.
Es werden deutlich aufgelöste Submikronstrukturen mit hoher Flankensteilheit erhalten.

### Beispiel 3:

2 g des Lösungsinhibitors gemäss Beispiel F werden zusammen mit 6 g Resin M® P4HS und 0,2 g Triphenylsulfoniumtrifluoromethansulfonat in 15 ml Cyclopentanon gelöst. Nach dem Applizieren auf eine Siliciumscheibe analog Beispiel 1 wird ein 0,9 µm dicker Film erhalten. Dieser Film wird bei 254 nm mit einer Belichtungsdosis von 7 mJ/cm² durch eine Maske belichtet und anschliessend während 60 Sekunden bei 90 °C erwärmt. Die so gewonnenen latenten Bilder werden in MF 312, verdünnt mit 4 Teilen Wasser, während 140 Sekunden entwickelt.
Es werden deutlich aufgelöste Submikronstrukturen mit hoher Flankensteilheit erhalten.

## Patentansprüche

1. Verbindungen der Formel I 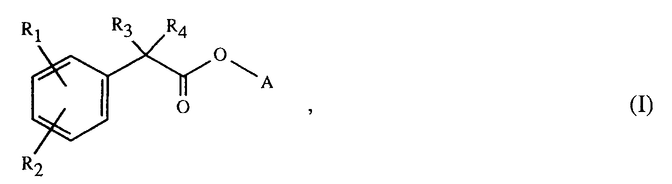 worin A einen Rest der Formeln 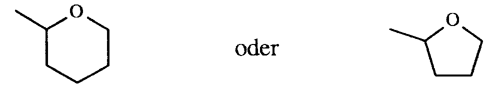 bedeutet,
R₁ und R₂ unabhängig voneinander Wasserstoff, C₁-C₄-Alkyl, C₁-C₄-Alkoxy, Halogen oder einen Rest der Formeln -CH₂-COOA oder 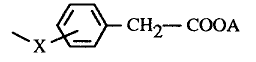 bedeuten, wobei X -O-, -CH₂-, -C(CH₃)₂- oder -SO₂- darstellt, und
R₃ und R₄ unabhängig voneinander Wasserstoff, C₁-C₄-Alkyl oder Phenyl bedeuten, wobei (2-Tetrahydrofuranyl)diphenylacetat und (2-Tetrahydropyranyl)diphenylacetat ausgeschlossen sind.

2. Verbindungen der Formel I gemäss Anspruch 1, worin R₂ Wasserstoff bedeutet.

3. Verbindungen der Formel I gemäss Anspruch 1, worin R₁ Wasserstoff, Halogen oder einen Rest der Formel -CH₂-COOA bedeutet, und R₃ und R₄ unabhängig voneinander Wasserstoff oder Phenyl bedeuten.

4. Verbindungen gemäss Anspruch 1, worin die Formel I die Formeln 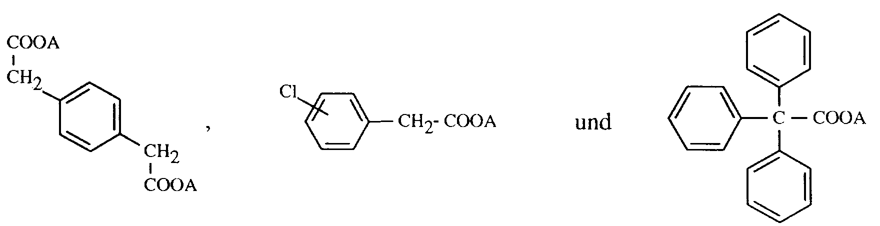 darstellt und A die in Anspruch 1 angegebene Bedeutung hat.

5. Strahlungsempfindliche Zusammensetzungen enthaltend, bezogen auf die Gesamtmenge der Komponenten A), B) und C),
A) 55 bis 95 Gew.% mindestens eines in wässrig-alkalischer Lösung löslichen Bindemittels,
B) 4,5 bis 40 Gew.% mindestens einer Verbindung gemäss Anspruch 1, von (2-Tetrahydrofuranyl)diphenylacetat oder von (2-Tetrahydropyranyl)diphenylacetat und
C) 0,5 bis 15 Gew.% mindestens einer unter Einwirkung aktinischer Strahlung säurebildenden Substanz.

6. Zusammensetzungen gemäss Anspruch 5 enthaltend, bezogen auf die Gesamtmenge der Komponenten A), B) und C),
A) 65 bis 90 Gew.% mindestens eines in wässrig-alkalischer Lösung löslichen Bindemittels,
B) 9 bis 30 Gew.% mindestens einer Verbindung gemäss Anspruch 1, von (2-Tetrahydrofuranyl)diphenylacetat oder von (2-Tetrahydropyranyl)diphenylacetat und
C) 1 bis 10 Gew.% mindestens einer unter Einwirkung aktinischer Strahlung säurebildenden Substanz.

7. Zusammensetzungen gemäss Anspruch 5, worin als Komponente A) ein phenolisches Harz eingesetzt wird.

8. Zusammensetzungen gemäss Anspruch 5, worin als Komponente A) ein Novolak, ein Poly-(4-hydroxystyrol), Poly-(4-hydroxy-α-methylstyrol), Copolymere von N-(4-Hydroxyphenyl)-maleinimid mit Styrol, 4-Hydroxystyrol, Acrylsäure oder Metharylsäure oder Copolymere von 4-Hydroxystyrol und Alkoxystyrol eingesetzt werden.

9. Zusammensetzungen gemäss Anspruch 5, worin als Komponente A) ein Poly-(4-hydroxystyrol) eingesetzt wird.

10. Verfahren zur Herstellung von positiven Abbildungen durch
I) Beschichtung eines Substrates mit einer strahlungsempfindlichen Zusammensetzung gemäss Anspruch 5,
II) Belichtung des beschichteten Substrates mit einem vorbestimmten Muster aktinischer Strahlung und
III) Entwicklung des belichteten Substrates.

11. Verwendung der Verbindungen gemäss Anspruch 1 als Lösungsinhibitoren in Photoresist-Zusammensetzungen.

12. Verwendung der Zusammensetzungen gemäss Anspruch 5 als Positiv-Resist für die Herstellung von Druckformen, gedruckten Schaltungen oder integrierten Schaltkreisen, sowie für silberlose photographische Filme.

13. Die unter Verwendung der Zusammensetzungen gemäss Anspruch 5 hergestellten Druckformen, gedruckten Schaltungen, integrierten Schaltkreise oder silberlosen photographischen Filme.

## Claims

1. Compounds of formula I 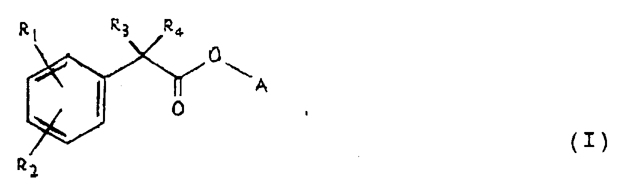 in which A is designates a residue of the formulae 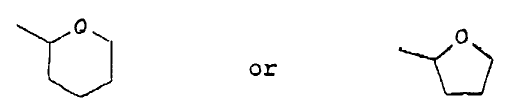 R₁ and R₂ designate, independently of one another, hydrogen, C₁-C₄-alkyl, C₁-C₄-alkoxy, halogen or a residue of the formulae -CH₂-COOA or 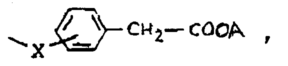 wherein X represents -O-, -CH₂-, -C(CH₃)₂- or -SO₂-, and
R₃ and R₄ designate, independently of one another, hydrogen, C₁-C₄-alkyl or phenyl, in wherein (2-tetrahydrofuranyl)diphenylacetate and (2-tetrahydropyranyl)diphenylacetate are excluded.

2. Compounds of formula I according to Claim 1, in which R₂ designates hydrogen.

3. Compounds of formula I according to Claim 1, in which R₁ designates hydrogen, halogen or a residue of the formula -CH-COOA, and R₃ and R₄ designate, independently of one another, hydrogen or phenyl.

4. Compounds according to Claim 1, in which formula I represents formulae 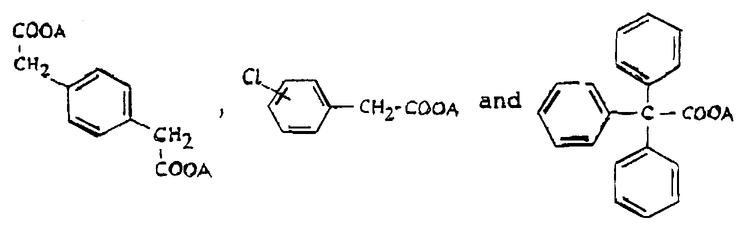 and A has the meaning given in Claim 1.

5. Radiosensitive compounds containing, in relation to the total amount of the components A), B), and C)
A) 55 to 95 weight % of at least one binder which is soluble in an aqueous-alcoholic solution;
B) 4.5 to 40 weight % of at least one compound according to Claim 1, of (2-tetrahydrofuranyl)diphenylacetate or of (2-tetrahydropyranyl)diphenylacetate; and
C) 0.5 of 15 weight % at at least one substance which is acid-forming under the effect of actinic radiation.

6. Compounds according to Claim 5, containing, in relation to the total amount of the components A) B) and C),
A) 65 to 90 weight % of at least one binder agent which is soluble in aqueous-alcoholic solution;
B) 9 to 30 weight % of at least one compound according to Claim 1, of (2-tetrahydrofuranyl)diphenylacetate or of (2-tetrahydropyranyl)diphenylacetate and
C) 1 to 10 weight % of at least one substance which is acid-forming under the effect of actinic radiation,

7. Compounds according to Claim 5, in which a phenolic resin is used as component A).

8. Compounds according to Claim 5, in which a novolak, a poly-(4-hydroxystyrene), poly-(4-hydroxy-α-methylstyrene), copolymers of N-(4-hydroxyphenyl)-maleimide with styrene, 4-hydroxystyrene, acrylic acid or methacrylic acid or copolymers of 4-hydroxystyrene and alkoxystyrene are used.

9. Compounds according to Claim 5, in which a poly-(4-hydroxystyrene) is used as component A).

10. Process for the production of positive images by means of
I) coating a substrate with a radiosensitive composition according to Claim 5;
II) illuminating the coated substrate with a predetermined pattern of actinic radiation; and
III) developing the illuminated substrate.

11. Application of the compounds according to Claim 1 as dissolution inhibitors in photoresist compounds.

12. Application of the compounds according to Claim 5 as a positive resists for the production of printing plates, printed circuits or integrated circuits, and for silver-free photographic films.

13. The printing plates, printed circuits, integrated circuits or silver-free photographic films produced using the compounds according to Claim 5.

## Revendications

1. Composés de formule I : 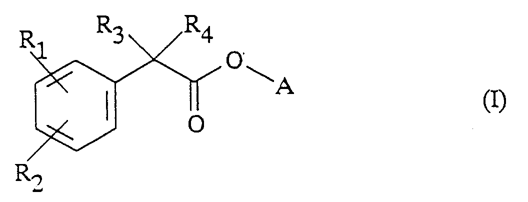 dans laquelle A représente un résidu de formule : 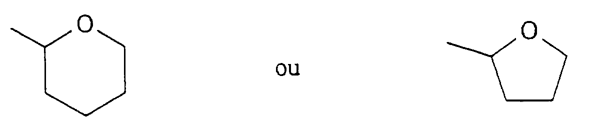 R₁ et R₂ représentent indépendamment l'un de l'autre l'hydrogène, un alkyle en C₁-C₄, un alcoxy en C₁-C₄, un halogène ou un résidu de formule -CH₂-COOA ou 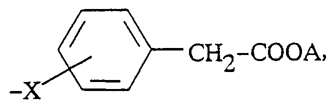 étant entendu que X représente -O-, -CH₂-, -C(CH₃)₂- ou -SO₂-, et
R₃ et R₄ représentent indépendamment l'un de l'autre l'hydrogène, un alkyle en C₁-C₄ ou le phényle, étant entendu que sont exclus le diphénylacétate de 2-tétrahydrofuranyle et le diphénylacétate de 2-tétrahydropyranyle.

2. Composés de formule I selon la revendication 1, pour lesquels R₂ représente l'hydrogène.

3. Composés de formule I selon la revendication 1, pour lesquels R₁ représente l'hydrogène,un halogène ou un résidu de formule -CH₂-COOA et R₃ et R₄ représentent indépendamment l'un de l'autre l'hydrogène ou le phényle.

4. Composés selon la revendication 1, pour lesquels la formule I représente les formules : 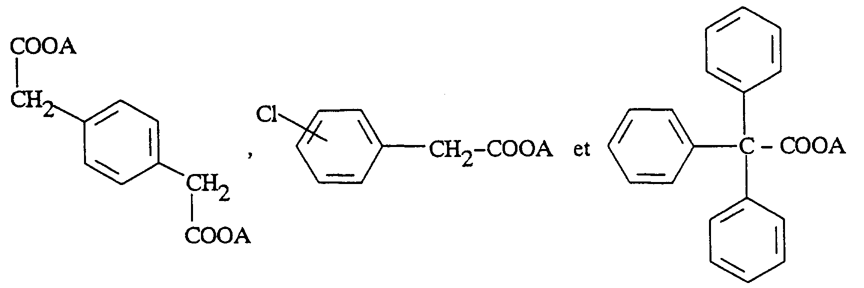 et A est tel que défini dans la revendication 1.

5. Compositions photosensibles contenant, par rapport à la quantité totale des composants A), B) et C),
A) de 55 à 95 % en poids d'au moins un liant soluble dans une solution aqueuse-alcaline,
B) de 4,5 à 40 % en poids d'au moins un composé selon la revendication 1, de diphénylacétate de 2-tétrahydrofuranyle ou de diphénylacétate de 2-tétrahydropyranyle et
C) de 0,5 à 15 % en poids d'au moins une substance acide sous l'action d'un rayonnement actinique.

6. Compositions selon la revendication 5 contenant, par rapport à la quantité totale des composants A), B) et C),
A) de 65 à 90 % en poids d'au moins un liant soluble dans une solution aqueuse-alcaline,
B) de 9 à 30 % en poids d'au moins un composé selon la revendication 1, de diphénylacétate de 2-tétrahydrofuranyle ou de diphénylacétate de 2-tétrahydropyranyle et
C) de 1 à 10 % en poids d'au moins une substance acide sous l'action d'un rayonnement actinique.

7. Compositions selon la revendication 5, dans lesquelles on utilise une résine phénolique en tant que composant A).

8. Compositions selon la revendication 5, dans lesquelles on utilise en tant que composant A) une résine novolaque, un poly-(4-hydroxystyrène), un poly-(4-hydroxy-α-méthylstyrène), les copolymères de N-(4-hydroxyphényl)-maléinimide et de styrène, le 4-hydroxystyrène, l'acide acrylique ou l'acide méthacrylique ou les copolymères de 4-hydroxystyrène et d'alcoxystyrène.

9. Compositions selon la revendication 5, dans lesquelles on utilise en tant que composant A) un poly-(4-hydroxystyrène).

10. Procédé de production d'images positives par :
I) revêtement d'un substrat par une composition photosensible selon la revendication 5,
II) exposition du substrat revêtu avec un dessin prédéfini de rayonnement actinique et
III) développement du substrat exposé.

11. Utilisation des composés selon la revendication 1 en tant qu'inhibiteurs de dissolution dans des compositions photorésists.

12. Utilisation des compositions selon la revendication 5 en tant que résist positif pour la production de clichés, de circuits imprimés ou de circuits intégrés, ainsi que pour des films photographiques sans argent.

13. Clichés, circuits imprimés, circuits intégrés ou films photographiques sans argent produits en utilisant les compositions selon la revendication 5.
